# EUROPEAN PATENT APPLICATION

(11) **EP 3 821 845 A1**
(43) Date of publication of application: **19.05.2021**
(21) Application number: 19834906.0
(22) Date of filing: 09.07.2019
(51) Int. Cl.: A61B 90/00, A61B 17/15, A61F 2/46, A61B 17/02

(54) **GAP GAUGE AND UNIT CAPABLE OF CHECKING FLEXION GAP PRIOR TO CUTTING OF POSTERIOR FEMUR**

(30) Priority: 10.07.2018 KR 20180080012
(71) Applicant: Corentec Co., Ltd., Cheonan-si, Chungcheongnam-do 31056 (KR)
(72) Inventor: OH, Seung Hun, Seoul 03691 (KR)
(74) Representative: Witte, Weller & Partner Patentanwälte mbB
(86) International application number: PCT/KR2019/008446
(87) International publication number: WO 2020/013585

(57) **Abstract**

The present disclosure relates to total knee arthroplasty used for a knee joint including a tibia and a femur of a patient. More particularly, the present disclosure relates to a gap gauge and a gap gauge unit capable of checking a flexion gap before femur posterior cutting, wherein the gap gauge includes a measurement unit configured to be capable of measuring a gap between a femur posterior cutting surface and a tibia proximal cutting surface during artificial knee joint surgery, wherein the measurement unit is coupled to and positioned in a slot in a femur posterior cutting guide placed on the femur posterior cutting surface so as to check a flexion gap before femur posterior cutting, wherein the measurement unit includes a first measurement unit placed between a tibia proximal cutting surface and the cutting guide before the femur posterior cutting so as to measure the flexion gap, wherein the first measurement unit includes a cutting slot coupling unit coupled to the slot in the cutting guide and a tibia seating portion seated on the tibia proximal cutting surface, and wherein the cutting slot coupling unit includes a blade configured to capable of being coupled to the cutting slot in the cutting guide and inserted into the cutting slot in the cutting guide, and a cutting block reception groove configured to receive a lower portion of the cutting guide during insertion of the blade so as to secure an insertion depth of the gap gauge.

## Description

### Technical Field

The present disclosure relates to total knee arthroplasty used for a knee joint including a tibia and a femur of a subject to be treated. More particularly, the present disclosure relates to a gap gauge and a gap gauge unit capable of checking a flexion gap before femur posterior cutting, wherein the gap gauge includes a measurement unit configured to be capable of measuring a gap between a femur posterior cutting surface and a tibia proximal cutting surface during artificial knee joint surgery, wherein the measurement unit is coupled to and positioned in a slot in a femur posterior cutting guide placed on the femur posterior cutting surface so as to check the flexion gap before femur posterior cutting, wherein the measurement unit includes a first measurement unit placed between a tibia proximal cutting surface and the cutting guide before the femur posterior cutting so as to measure the flexion gap, wherein the first measurement unit includes a cutting slot coupling unit coupled to the slot in the cutting guide and a tibia seating portion seated on the tibia proximal cutting surface, and wherein the cutting slot coupling unit includes a blade configured to be capable of being coupled to the cutting slot in the cutting guide and inserted into the cutting slot in the cutting guide, and a cutting block reception groove configured to receive a lower portion of the cutting guide during insertion of the blade so as to secure an insertion depth of the gap gauge.

### Background Art

A knee joint is a joint formed by a femur, a tibia, and a patella, which are the three bones surrounding the knee, and corresponds to a key joint related to movement using legs, such as walking or running using joint movements and supporting the weight of a person.

An articular cartilage is present at the end of a femur, and a meniscus is present at the end of a tibia. When the cartilage is damaged due to extreme exercise, aging, or the like, bones may come into direct contact with each other, which may cause severe pain.

Total knee arthroplasty is a surgery in which a part of a femur and a tibia is incised and an artificial knee joint is inserted when a knee joint is damaged. A surgical procedure for an artificial knee joint is performed by connecting a femur joint member to the end of the femur, fixedly inserting a tibia element into the end of the tibia, and installing a bearing member thereon.

Conventionally, such a total knee arthroplasty is generally performed through the following process.

Surgical site incision and exposure -> femur distal cutting -> tibia proximal cutting -> extension gap measurement and balancing -> femur size and angle measurement and balancing -> femur anterior, posterior, or corner cutting -> flexion gap measurement -> tibia member test -> patella cutting -> final component implant

As can be seen from the above-described process, conventionally, there was no instrument that is capable of accurately measuring a flexion gap before cutting. Therefore, there was no choice but to use a method of measuring and checking the flexion gap using a gap gauge or a separate device after femur posterior cutting. However, this method has a problem in that once wrong cutting is performed, an irreversible problem occurs. In this case, a situation arises in which it is necessary to proceed with the cutting again or to proceed with the surgery by adjusting the remaining members while leaving the cut surface as it is. Accordingly, an incorrect implant may be implanted, which may cause the patient's discomfort and pain, and the risk of revision surgery. Furthermore, when a cutting error is severe, there is a problem in that the tibia member prepared before the surgery does not fit, resulting in a situation in which it is impossible to proceed with surgery itself.

In order to alleviate this problem, there has been proposed a method of measuring a flexion gap in advance before femur posterior cutting by forming a new groove other than the cutting slot in the cutting guide attached to the femur distal cutting surface and introducing a new instrument for measuring a gap before cutting. This method also has a problem in that the gap gauge used after cutting and the measurement gauge used before cutting are configured separately, and thus the number of instruments used for surgery increases significantly, which impairs the convenience of surgery, and in that an economic burden is added because it is necessary to purchase new instruments in addition to the existing generally compatible surgical instruments.

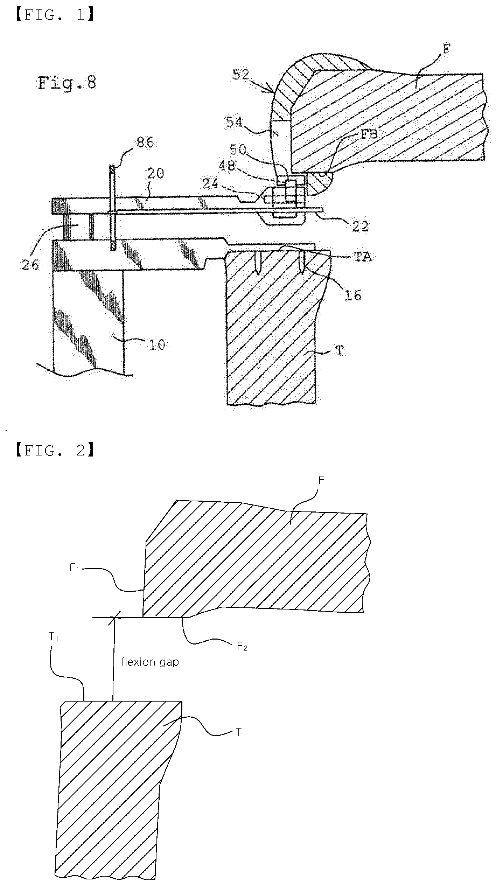

U.S. Patent No. 7,156,853 B2 discloses a flexion gap measurement device illustrated in FIG. 1. Referring to FIG. 1, the flexion gap measurement device is a device capable of measuring an extension gap and a flexion gap after femur distal cutting and femur posterior cutting.

However, as pointed out in the foregoing description, it is impossible to check a flexion gap before femur posterior cutting using the gap measurement instrument of the above-mentioned patent, and thus when the cutting is wrong during the surgical procedure, an irreversible problem occurs. In particular, in addition to the problems caused by a wrongly cut surface, it may be necessary to completely recalibrate the numerical values of the tibia implant member.

Therefore, the necessity of introducing a gap gauge that enables accurate knee arthroplasty by checking a flexion gap before femur posterior cutting without adding new instruments or surgical procedures is increasing.

### Detailed Description of the Invention

### Technical Problem

The present disclosure has been conceived in order to solve the problems described above.

An objective of the present disclosure is to provide a gap gauge, wherein the gap gauge includes a measurement unit configured to enable measurement of a gap between a femur posterior cutting surface and a tibia proximal cutting surface, and the measurement unit is coupled to and positioned in a slot in a femur posterior cutting guide placed on the femur posterior cutting surface so as to check a flexion gap before femur posterior cutting, thereby enabling accurate knee replacement surgery and preventing side effects and reoperation.

Another objective of the present disclosure is to provide a gap gauge, wherein the measurement unit includes a first measurement unit placed between the tibia proximal cutting surface and the cutting guide before the femur posterior cutting so as to measure the flexion gap, thereby enabling measurement of a flexion gap before the cutting procedure.

Another objective of the present disclosure is to provide a gap gauge, wherein the first measurement unit includes a cutting slot coupling unit coupled to the slot in the cutting guide and a tibia seating portion seated on the tibia proximal cutting surface so that the gap gauge can be stably fixed to the cutting guide and the tibia cutting surface, thereby enabling accurate measurement during surgery.

Another objective of the present disclosure is to provide a gap gauge, wherein the cutting slot coupling unit is configured to be coupled to the cutting slot in the cutting guide so that the cutting slot of the existing cutting guide is used, whereby the gap gauge is compatible with existing equipment without introducing new surgical systems or modifying the existing surgical instruments.

Another objective of the present disclosure is to provide a gap gauge, wherein the cutting slot coupling unit includes a blade configured to be inserted into the cutting slot in the cutting guide and a cutting block reception groove configured to receive a lower portion of the cutting guide during insertion of the blade so as to secure an insertion depth of the gap gauge so that the cutting slot coupling unit can be inserted into the cutting guide to a sufficient depth, whereby it is possible to secure a predetermined fixing force and to minimize errors during the gap measurement.

Another objective of the present disclosure is to provide a gap gauge, wherein the blade is made of a metal in order to secure the strength of the blade during the flexion gap measurement, thereby preventing breakage of a thin blade during surgery.

Another objective of the present disclosure is to provide a gap gauge, wherein the cutting slot coupling unit includes a fixing means for fixing the blade to the first measurement unit so as to secure a fixing force between the blade made of the metal and a remaining unit, which may be made of another material, and to prevent displacement or separation of the blade, thereby enabling stable and accurate gap measurement during surgery.

Another objective of the present disclosure is to provide a gap gauge, wherein the gap gauge includes a second measurement unit configured to measure an extension gap after femur distal cutting, and the measurement gauge used before cutting and the measurement gauge used after cutting are integrally configured, whereby the number of instruments used in surgery can be significantly reduced.

Another objective of the present disclosure is to provide a gap gauge, wherein each of the first measurement unit and the second measurement unit includes a groove capable of receiving a cruciate ligament of a subject to be treated, whereby it is possible to prevent damage to a posterior cruciate ligament around the surgical site during a gap measurement process.

Another object of the present disclosure is to provide a gap gauge unit including a cutting guide located on a femur distal cutting surface, whereby it is possible to check a flexion gap before femur posterior cutting.

### Technical Solution

In order to achieve the objects of the present disclosure, the present disclosure is implemented by embodiments having features as follows.

According to an embodiment of the present disclosure, a gap gauge includes a measurement unit configured to measure a gap between a femur posterior cutting surface and a tibia proximal cutting surface, wherein the measurement unit is coupled to and positioned in a slot in a femur posterior cutting guide placed on the femur posterior cutting surface so as to check a flexion gap before femur posterior cutting.

According to another embodiment of the present disclosure, the measurement unit includes a first measurement unit placed between a tibia proximal cutting surface and the cutting guide before the femur posterior cutting so as to measure the flexion gap.

According to another embodiment of the present disclosure, the first measurement unit includes a cutting slot coupling unit coupled to the slot in the cutting guide and a tibia seating portion seated on the tibia proximal cutting surface.

According to another embodiment of the present disclosure, the cutting slot coupling unit is configured to be coupled to the cutting slot in the cutting guide.

According to another embodiment of the present disclosure, the cutting slot coupling unit includes a blade configured to be inserted into the cutting slot in the cutting guide and a cutting block reception groove configured to receive a lower portion of the cutting guide during insertion of the blade so as to secure an insertion depth of the gap gauge.

According to another embodiment of the present disclosure, the blade is made of a metal so as to secure strength of the blade during flexion gap measurement.

According to another embodiment of the present disclosure, the cutting slot coupling unit includes a fixing means configured to fix the blade to the first measurement unit.

According to another embodiment of the present disclosure, the gap gauge includes a second measurement unit configured to measure the extension gap after femur distal cutting.

According to another embodiment of the present disclosure, each of the first measurement unit and the second measurement unit includes a groove capable of receiving a cruciate ligament of a subject to be treated.

According to another embodiment of the present disclosure, a gap gauge unit includes a cutting guide located on the femur distal cutting surface.

### Advantageous Effects

The present disclosure is capable of obtaining the following effects through a combination of the above-described embodiments and the configurations to be described below and a use relationship therebetween.

According to the present disclosure, the gap gauge includes a measurement unit configured to enable measurement of a gap between a femur posterior cutting surface and a tibia proximal cutting surface, and the measurement unit is coupled to and positioned in a slot in a femur posterior cutting guide placed on the femur posterior cutting surface so as to check a flexion gap before femur posterior cutting. As a result, it is possible to perform accurate knee replacement surgery and to prevent side effects and reoperation.

According to the present disclosure, the measurement unit is placed between the tibia proximal cutting surface and the cutting guide before the femur posterior cutting so as to measure the flexion gap.

According to the present disclosure, the first measurement unit includes a cutting slot coupling unit coupled to the slot in the cutting guide, and a tibia seating portion seated on the tibia proximal cutting surface so as to stably fix the gap gauge to the cutting guide and the tibia cutting surface. Therefore, it is possible to perform accurate measurement of a flexion gap during surgery.

According to the present disclosure, the cutting slot coupling unit is configured to be coupled to the cutting slot in the cutting guide so that the cutting slot of the existing cutting guide is used. Therefore, the gap gauge is compatible with existing equipment without introducing new surgical systems or modifying the existing surgical instruments.

According to the present disclosure, the cutting slot coupling unit includes a blade configured to be inserted into the cutting slot in the cutting guide and a cutting block reception groove configured to receive a lower portion of the cutting guide during insertion of the blade so as to secure an insertion depth of the gap gauge so that the cutting slot coupling unit can be inserted into the cutting guide to a sufficient depth. Therefore, it is possible to secure a predetermined fixing force and to minimize errors during the gap measurement.

According to the present disclosure, the blade is made of a metal in order to secure the strength of the blade during the flexion gap measurement. Therefore, it is possible to prevent breakage of a thin blade during surgery.

According to the present disclosure, the cutting slot coupling unit includes a fixing means for fixing the blade to the first measurement unit so as to secure a fixing force between the blade made of the metal and a remaining unit, which may be made of another material, and to prevent displacement or separation of the blade. Therefore, it is possible to stably and accurately measure a gap during surgery.

According to the present disclosure, the gap gauge includes a second measurement unit configured to measure an extension gap after femur distal cutting, and the measurement gauge used before cutting and the measurement gauge used after cutting are integrally configured. Therefore, it is possible to significantly reduce the number of instruments used in surgery.

According to the present disclosure, each of the first measurement unit and the second measurement unit includes a groove capable of receiving a cruciate ligament of a subject to be treated. Therefore, it is possible to prevent damage to a posterior cruciate ligament around the surgical site during a gap measurement process.

According to the present disclosure, the gap gauge unit includes a cutting guide located on a femur distal cutting surface. Therefore, it is possible to check a flexion gap before the femur posterior cutting.

### Brief Description of the Drawings

FIG. 1 is a view illustrating an embodiment of a conventional gap measurement device for use in total knee arthroplasty.
FIG. 2 is a cross-sectional view illustrating femur and tibia cutting surfaces and a flexion gap.
FIG. 3 is a perspective view illustrating a gap gauge and a gap gauge unit according to an embodiment of the present disclosure that is capable of checking a flexion gap before femur posterior cutting, wherein the gap gauge and the gap gauge unit are viewed before the gap gauge is coupled to a cutting guide.
FIG. 4 is a perspective view illustrating a gap gauge and a gap gauge unit according to another embodiment of the present disclosure that is capable of checking a flexion gap before femur posterior cutting, wherein the gap gauge and the gap gauge unit are viewed after the gap gauge is coupled to a cutting guide.
FIG. 5 is an exploded perspective view illustrating a gap gauge according to another embodiment of the present disclosure that is capable of checking a flexion gap before femur posterior cutting.
FIG. 6 is a perspective view illustrating a gap gauge according to another embodiment of the present disclosure that is capable of checking a flexion gap before femur posterior cutting.
FIG. 7 is a side view illustrating a gap gauge of the present disclosure that is capable of checking a flexion gap before femur posterior cutting.
FIG. 8 is a cross-sectional view taken along line A-A'in FIG. 3.

### Mode for Carrying Out the Invention

Hereinafter, with reference to the accompanying drawings, a gap gauge and a gap gauge unit according to the present disclosure that are capable of checking a flexion gap before femur posterior cutting will be described in detail. It shall be noted that the same elements in the drawings are denoted by the same reference numerals if possible. In the following description, a detailed description of a well-known function or construction will be omitted when it may make the subject matter of the present disclosure unnecessarily unclear. Unless defined otherwise, all terms used herein have the same meaning as the general meaning of the terms understood by a person ordinarily skilled in the art to which this disclosure belongs and, when the general meaning is in conflict with the meaning of the terms used herein, the meaning of the terms follows the definition used in the specification.

In describing the body part of a subject to be treated, the side closer to the torso is referred to as a "body" or "proximal" side, and, on the contrary, the side far from the torso is referred to as a "distal" side. In addition, the front side of the body of a subject to be treated is referred to as an "anterior" side, and, on the contrary, the rear side is referred to as a "posterior" side. In addition, an imaginary line that evenly divides the left and right sides of a subject to be treated is referred to as a midline, the side facing the midline is called a "medial" side, and the side facing away from the midline is called a "lateral" side.

FIG. 2 is a view illustrating cut surfaces of a femur F and a tibia T and a flexion interval therebetween in total knee arthroplasty. A femur of a subject to be treated is denoted by F, a femur distal cutting surface is denoted by F1, a femur posterior cutting surface is denoted by F2, a tibia is denoted by T, and a tibia proximal cutting surface is denoted by T1.

While an "extension gap" represents the spacing between the femur distal cutting surface F1 and the tibia proximal cutting surface T1 when the femur and tibia are in a straight line, a "flexion gap" represents the spacing between the femur posterior cutting surface F2 and the tibia proximal cutting surface T1 when the femur and tibia are bent or form an angle of about 90 degrees. The extension gap and the flexion gap are measured before and after femur posterior cutting during total knee arthroplasty in order to obtain values thereof required for reproducing normal joint movements of the subject to be treated after the total knee arthroplasty.

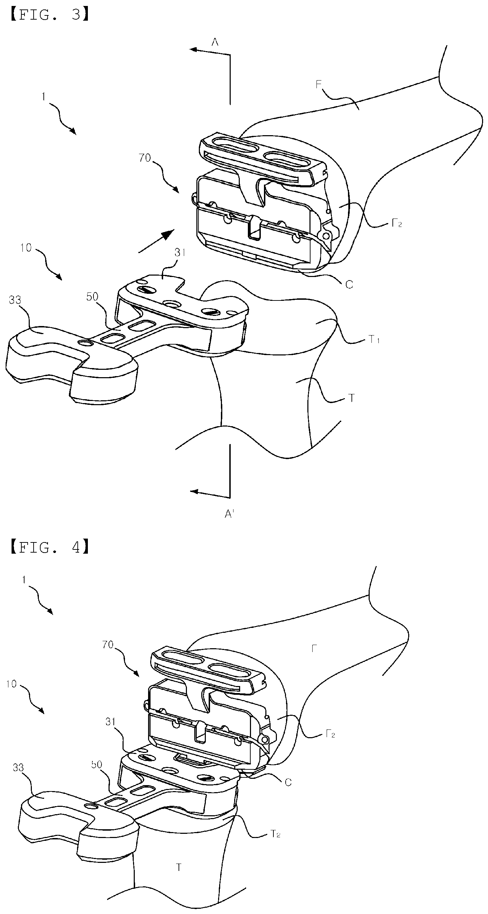

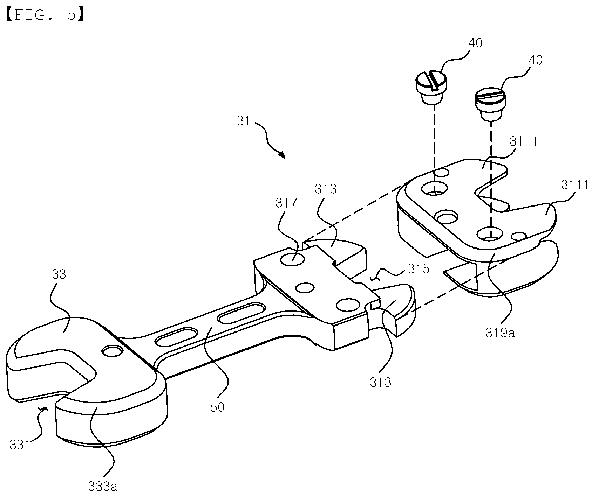

FIG. 3 is a perspective view illustrating a gap gauge and a gap gauge unit according to an embodiment of the present disclosure that is capable of checking a flexion gap before femur posterior cutting, wherein the gap gauge and the gap gauge unit are viewed before the gap gauge is coupled to a cutting guide, FIG. 4 is a perspective view illustrating a gap gauge and a gap gauge unit according to another embodiment of the present disclosure that is capable of checking a flexion gap before femur posterior cutting, wherein the gap gauge and the gap gauge unit are viewed after the gap gauge is coupled to a cutting guide, and FIG. 5 is an exploded perspective view illustrating a gap gauge according to another embodiment of the present disclosure that is capable of checking a flexion gap before femur posterior cutting.

Referring to FIGS. 3 to 5, a gap gauge unit 1 of the present disclosure may include a gap gauge 10 and a cutting guide 70. The gap gauge 10 may include a measurement unit 30 and a connection unit 50. The measurement unit 30 may include a first measurement unit 31 and a second measurement unit 33.

The gap gauge 10 adopts a shape of a gourd-shaped bottle or a spanner similar to a gap gauge generally used for measuring a flexion gap and an extension gap after cutting a femur. Thus, with the gap gauge, it is possible to measure a gap before femur posterior cutting, and even after femur posterior cutting, to measure a flexion gap and an extension gap using a first measurement unit 31 or a second measurement unit 33 to be described later according to a conventional method of using a conventional gap gauge.

As described above, the first measurement unit 31 is formed at one end of the gap gauge 10 having a shape similar to a gourd-shaped bottle or a spanner, and may have a wide and flat pillar shape having rectangular top and bottom surfaces with rounded vertices. If necessary, since a U-shaped groove 315 is formed at the end of the first measurement unit 31, the first measurement unit 31 may have a shape similar to the shape of the head of the spanner. The first measurement unit 31 may include a configuration capable of measuring the flexion gap before femur posterior cutting, and as will be described later, by adopting the external appearance of the conventional gap gauge as it is, the first measurement unit 31 may include a configuration capable of measuring a flexion gap and an extension gap after femur distal cutting or femur posterior cutting.

As illustrated in FIGS. 3 to 5, the first measurement unit 31 may include a cutting slot coupling unit 311, a tibia seating portion 313, a groove 315, and a screw coupling portion 317, which will be inclined below. Preferably, as illustrated in FIGS. 3 to 5, the corner portions of the top and bottom surfaces of the first measurement unit 31 may respectively have, along the outer peripheries thereof, inclined portions 319a and 319b having a predetermined inclination in order to prevent damage to a soft tissue therearound.

As described above, the second measurement unit 33 is formed at the other end of the gap gauge 10 having a shape similar to a gourd-shaped bottle or a spanner, and may have a wide and flat pillar shape having rectangular top and bottom surfaces with rounded vertices. If necessary, since a U-shaped groove 331 is formed at the end of the second measurement unit 33, the second measurement unit 33 may have a shape similar to the shape of the head of the spanner. As will be described later, by adopting the external appearance of the conventional gap gauge as it is, the second measurement unit 33 is capable of measuring an extension gap and a flexion gap after femur distal cutting or femur posterior cutting according to a method of using the conventional gap gauge. Preferably, the second measurement unit 33 may further include a U-shaped groove 331 for receiving a posterior cruciate ligament of a subject to be treated when the gap gauge 10 is inserted. More preferably, as illustrated in FIGS. 3 to 5, the corner portions of the top and bottom surfaces of the second measurement unit 33 may respectively have, along the outer peripheries thereof, inclined portions 333a and 333b having a predetermined inclination in order to prevent damage to a soft tissue therearound.

The connection unit 50 is a rod-shaped component located in the middle of the gap gauge 10 and connecting the first measurement unit 31 and the second measurement unit 33. The connection unit 50 may serve as a handle for inserting or removing the gap gauge 10 while connecting both measurement units 30. Preferably, a hole for insertion of an alignment rod may be formed so as to provide a reference for a cutting surface by aligning the bones of a subject to be treated during surgery.

Referring to FIGS. 3 to 5, although there is a gap gauge 10 for measuring an extension gap and a flexion gap after cutting in a surgical procedure in the related art, there is no instrument for measuring a flexion gap before femur posterior cutting as in the present disclosure. Although there is a measurement method using a modified cutting guide 70 and new measurement instruments, it is impossible to use the existing conventional cutting guide 70 and instruments as they are. Further, there is a hassle in that it is necessary to newly add a surgical procedure or instruments.

However, when the measurement units 30 having different functions at both ends are made in a single instrument, it is possible to use the conventional cutting guide 70 as it is and to maintain the shape and use method of the conventional gap gauge 10 as they are. Thus, it is possible to improve convenience of surgery and significantly reduce the number of instruments required during surgery. Accordingly, in the present disclosure, the first measurement unit 31 is formed at one end of the gap gauge 10, a second measurement unit 33 is formed at the other end of the gap gauge 10, and a connection part 50 disposed between and connecting the first and second measurement units 30 so as to form a single surgical instrument. Therefore, it is possible to select an appropriate measurement unit 30 according to the type of measurement required in the surgical procedure so as to measure a gap between bones and to determine a tibia member suitable for the measured gap.

In addition, as illustrated in FIGS. 3 and 4, the gap gauge 10 according to the present disclosure may be attached to the femur distal cutting surface F1 so as to form a single unit 1 with a cutting guide 70 configured to guide a cutting surface. A blade 3111 of the gap gauge is inserted into a slot in the cutting guide 70 attached to the femur distal cutting surface F1 of the subject to be treated, and the gap gage is located in the space between the bottom portion of the cutting guide 70 and the tibia proximal cutting surface T1 so as to measure a flexion gap. Preferably, as illustrated in FIG. 4, the blade 3111 is inserted into a femur posterior cutting slot C, which is generally formed in the existing cutting guide 70. Thus, it is possible to use the existing cutting guide 70 for measuring the flexion gap as it is without adding new surgical instruments or modifying the existing cutting guide 70.

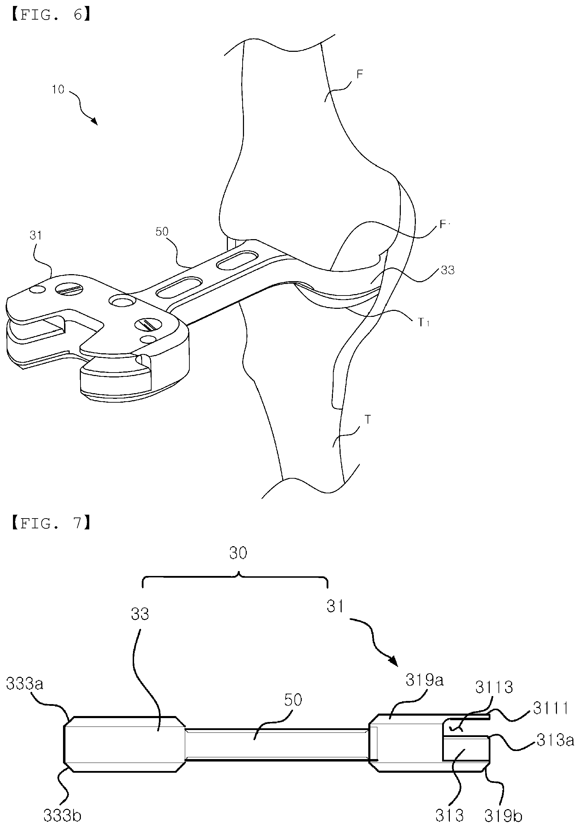

FIG. 6 is a perspective view illustrating a gap gauge according to another embodiment of the present disclosure that is capable of checking a flexion gap in the state in which an extension gap is being measured using a second measurement unit before femur posterior cutting.

Referring to FIG. 6, in the surgical procedure of total knee arthroplasty, flexion gaps or extension gaps of respective subjects to be treated are different from each other. Thus, in order to measure the gaps, gap gauges having various sizes are required. When an extension gap measurement device and a flexion gap measurement device are separately configured, the number of surgical instruments required in the surgical procedure relatively increases. However, as described above, the second measurement unit 33 of the present disclosure adopts the shape of the conventional gap gauge for measuring a gap after cutting a femur as it is, so that a required measurement unit 30 can be selected and used among the measurement units 30 in a surgery procedure. Therefore, it is possible to greatly reduce the number of surgical instruments. Therefore, the second measurement unit 33 can be used to measure an extension gap after femur posterior cutting according to a method of using the conventional gap gauge, as well as to measure a flexion gap after femur posterior cutting as in the prior art illustrated in FIG. 1. In addition, since the first measurement unit 31 also has a shape and configuration similar to the second measurement unit 33 except for the blade 3111 and the cutting guide reception groove 3113, it is also possible to use the first measurement unit 31 to measure a flexion gap before cutting as well as to measure a flexion gap and an extension gap after cutting.

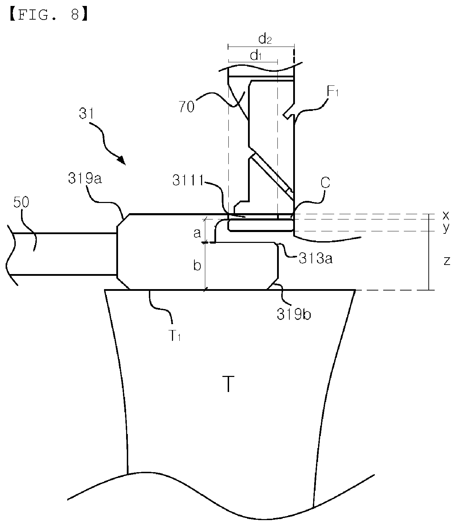

FIG. 7 is a side view illustrating a gap gauge according to the present disclosure that is capable of checking a flexion gap before femur posterior cutting, and FIG. 8 is a detailed cross-sectional view of the gap gauge 10 and the cutting guide 70 of the present disclosure that are capable of checking a flexion gap before femur posterior cutting, in the state in which the gap gauge 10 and the cutting guide 70 are coupled to each other.

In the following, "a" is the height from the bottom surface of the blade to the top surface of a tibia seating portion, "b"is the height from the top surface to the bottom surface of the tibia seating portion, "x" is the height of the cutting slot of the cutting guide, "y" is the height of the lower portion of the cutting guide, and "z" is the height from the bottom surface of the cutting guide to the tibia proximal cutting surface.

As illustrated in FIGS. 3 to 8, the first measurement unit 31 may include a cutting slot coupling unit 311, a tibia seating portion 313, a groove 315, and a screw coupling portion 317.

The cutting slot coupling unit 311 is configured to be coupled and fixed to the slot in the cutting guide 70 attached to the cutting surface after femur distal cutting, and is located at the upper end of the first measurement unit 31 having a shape of a wide square pillar. In order to secure the ease of insertion of the gap gauge 10, the side surface of the square pillar in the insertion direction among the corners of the wide square pillar may be formed in a round shape, and if necessary, the cutting slot coupling unit 311 may be provided with a U-shaped groove 315 to be described later and may thus have the same shape as the head of a spanner. The cutting slot coupling unit 311 may include a blade 3111 and a cutting guide reception groove 3113. Preferably, the slot in the cutting guide 70 may be a femur posterior cutting slot C formed in a cutting guide 70 that has been generally used in the past.

The blade 3111 has a thin plate-shaped configuration protruding from the upper end of the cutting slot coupling unit 311 in the insertion direction of the gap gauge 10, and is inserted into the cutting slot in the cutting guide 70 and serves to fix the gap gauge 10 while serving as a reference point for gap measurement. In general, the cutting slot C in the cutting guide 70 has a shape of a shallow and long groove, and thus, the blade 3111 to be inserted also has a thin and long shape, thereby enabling easy insertion. Preferably, since the blade 3111 is configured to be thin, there is a risk of damage or deformation during insertion in the surgical procedure. Thus, in order to prevent this, the blade 3111 may be made of a metal material. More preferably, a U-shaped groove 315 may be formed at the end of the blade 3111 to receive the posterior cruciate ligament located in the posterior femur portion of a subject to be treated. More preferably, the blade 3111 is provided in the form of a housing including the same, and the housing is provided in a hollow shape so as to receive the screw coupling portion 317 and the tibia seating portion 313 such that the upper side of the housing forms the blade 3111 and the lower side of the housing forms the bottom surface of the tibia seating portion 313. This is to make the blade 3111 have a shape that surrounds all or part of the outside of the first measurement unit 31 in order to securely couple the blade 3111 when the blade 3111 is made of a metal.

The cutting guide reception groove 3113 forms a recessed groove between the blade 3111 and the tibia seating portion 313 is configured to secure the insertion depth of the blade 3111 by preventing the lower portion of the cutting guide 70 and the end of the gap gauge 10 or the tibia seating portion 313 from colliding with each other when the blade 3111 is inserted into the cutting guide 70. Preferably, in order to prevent damage to the cutting surface of the distal tibia portion of a subject to be treated due to excessive insertion, the depth d1 of the cutting guide reception groove 3113 is shallower than the depth d2 of the cutting slot in the cutting guide 70, and in order to allow the lower portion of the cutting guide 70 to be sufficiently received, the height a from the bottom surface of the blade 3111 to the top surface of the tibia seating portion 313 may be formed to have a height greater than the height y from the cutting slot C to the bottom surface of the cutting guide 70.

The tibia seating portion 313 facilitates the insertion of the gap gauge 10 between the cutting surfaces of bones of a subject to be treated during the insertion of the gap gauge 10, secures a gap for flexion gap measurement between the bones, and stably fixes the gap gauge 10, thereby assisting in accurate gap measurement. Preferably, as illustrated in FIGS. 3 to 5, the edge portion of the top surface of the tibia seating portion 313 may have an inclined portion 313a having a predetermined inclination along the outer circumferential surface of the top surface of the tibia seating portion 313 in order to prevent damage to surrounding soft tissue during the insertion of the gap gauge.

The groove 315 is an empty space located in the middle of the end of the first measurement unit 31 and formed in a shape recessed toward the connection unit 50, and is configured to receive a posterior cruciate ligament present in the posterior femur portion of a subject to be treated during the insertion of the gap gauge 10 so as to prevent damage to the ligament. Preferably, in order to prevent damage to the ligament during the insertion of the gap gauge 10, the inside of the groove 315 may have a rounded U shape.

The screw coupling portion 317 is a portion formed on the top surface of the cutting slot coupling unit 311 or the first measurement unit 31 such that the blade 3111 is fixedly seated thereon. Since the blade 3111 may be made of a thin metallic material, a fixing means such as a screw is coupled to the screw coupling portion 317 to firmly fix the blade 3111 in order to secure the coupling force between the blade 3111 and the first measurement unit 31.

Referring to FIGS. 7 and 8, the first measurement unit 31 is located at one end of the gap gauge 10 of the present disclosure, and the blade 3111 is provided on the top side of the first measurement unit 31 and coupled to the cutting slot C in the cutting guide 70, thereby providing a reference point for measurement. The tibia seating portion 313 is located on the bottom side of the first measurement unit 31 so as to provide another reference point for gap measurement, and the cutting guide reception groove 3113 is located between the blade 3111 and the tibia seating portion 313 so as to receive the lower portion of the cutting guide 70 and to secure the insertion depth of the blade 3111. As described above, for ease of insertion, the height a from the bottom surface of the blade to the top surface of the tibial seating portion is preferably sufficiently greater than the height y of the lower portion of the cutting guide.

As illustrated in FIG. 7, the flexion gap is y+z, and when the gap (a+b) of the gap gauge 10 used for the first time does not match the flexion gap of a subject to be treated, the gap (a+b) of the gap gauge 10 that matches the flexion gap (y+z) of the subject to be treated can be found by selecting and testing gap gauges 10 having other heights or a gap (a+b) several times.

The foregoing detailed description exemplifies the present disclosure. In addition, the foregoing description is intended to illustrate and explain embodiments of the present disclosure, and the present disclosure may be used in various other combinations, modifications, and environments. That is, it is possible to change or modify the present disclosure within the scope of the concept of the present disclosure disclosed in this specification, within the scope equivalent to the above-described contents, and/or within the scope of the skills or knowledge in the art. The embodiments described above are intended to illustrate the best mode for carrying out the technical idea of the present disclosure, and various modifications required for specific applications and uses of the present disclosure are also possible. Therefore, the detailed description of the present disclosure is not intended to limit the present disclosure to the disclosed embodiments. In addition, the appended claims should be interpreted as covering other embodiments as well.

## Claims

1. A gap gauge comprising:
a measurement unit configured to be capable of measuring a gap between a femur posterior cutting surface and a tibia proximal cutting surface,
wherein the measurement unit is coupled to and positioned in a slot in a femur posterior cutting guide placed on the femur posterior cutting surface so as to check a flexion gap before femur posterior cutting.

2. The gap gauge of claim 1, wherein the measurement unit comprises a first measurement unit placed between a tibia proximal cutting surface and the cutting guide so as to measure the flexion gap before the femur posterior cutting.

3. The gap gauge of claim 2, wherein the first measurement unit comprises a cutting slot coupling unit coupled to the slot in the cutting guide and a tibia seating portion seated on the tibia proximal cutting surface.

4. The gap gauge of claim 3, wherein the cutting slot coupling unit is configured to be coupled to the cutting slot in the cutting guide.

5. The gap gauge of claim 4, wherein the cutting slot coupling unit comprises a blade configured to be inserted into the cutting slot in the cutting guide and a cutting block reception groove configured to receive a lower portion of the cutting guide during insertion of the blade so as to secure an insertion depth of the gap gauge.

6. The gap gauge of claim 5, wherein the blade is made of a metal so as to secure strength of the blade during flexion gap measurement.

7. The gap gauge of claim 6, wherein the cutting slot coupling unit comprises fixing means configured to fix the blade to the first measurement unit.

8. The gap gauge of claim 7, wherein the gap gauge comprises a second measurement unit configured to measure an extension gap after femur distal cutting.

9. The gap gauge of claim 8, wherein each of the first measurement unit and the second measurement unit comprises a groove capable of receiving a cruciate ligament of a subject to be treated.

10. A gap gauge unit of any of claims 1 to 9, comprising a cutting guide located on a femur distal cutting surface.
